# EUROPEAN PATENT SPECIFICATION

(11) **EP 3 996 673 B1**
(45) Date of publication and mention of the grant of the patent: **27.09.2023**
(21) Application number: 20735622.1
(22) Date of filing: 07.07.2020
(51) Int. Cl.: A61K 9/06, A61K 9/00, A61K 47/02, A61P 19/00, A61K 31/4168, A61K 31/728, A61K 33/06, A61K 35/16, A61L 27/36, A61L 27/52, A61L 27/54

(54) **A METHOD FOR THE PREPARATION OF A GEL-FORMING COMPOSITION**
VERFAHREN ZUR HERSTELLUNG EINER GELBILDENDEN ZUSAMMENSETZUNG
PROCÉDÉ DE PRÉPARATION D'UNE COMPOSITION DE FORMATION DE GEL

(30) Priority: 08.07.2019 EP 19184936
(43) Date of publication of application: 18.05.2022
(73) Proprietor: TheraVet SA, 6041 Charleroi (BE)
(72) Inventor: BASTIANELLI, Enrico, 1640 Rhode-Saint-Genèse (BE); WINAND, Julie, 5024 Marche les Dames (BE); ENA, Sabrina, 6110 Montigny le Tilleul (BE); ZEIPPEN, Caroline, 5370 Flostoy (BE)
(74) Representative: Brantsandpatents bv
(86) International application number: PCT/EP2020/069142
(87) International publication number: WO 2021/005069

(56) References cited:
- WO-A1-2014/035721
- WO-A2-2009/016451
- CN-A- 104 587 525
- US-A1- 2012 171 179
- MUN-IK LEE ET AL: "A placebo-controlled study comparing the efficacy of intra-articular injections of hyaluronic acid and a novel hyaluronic acid-platelet-rich plasma conjugate in a canine model of osteoarthritis", JOURNAL OF ORTHOPAEDIC SURGERY AND RESEARCH, vol. 14, no. 1, 18 September 2019 (2019-09-18), XP055655871, DOI: 10.1186/s13018-019-1352-1
- FABRIZIO RUSSO ET AL: "Platelet Rich Plasma and Hyaluronic Acid Blend for the Treatment of Osteoarthritis: Rheological and Biological Evaluation", PLOS ONE, vol. 11, no. 6, 16 June 2016 (2016-06-16), pages 1-12, XP55625964, DOI: 10.1371/journal.pone.0157048
- Pietro Gentile ET AL: "Use of Platelet Rich Plasma and Hyaluronic Acid in the Treatment of Complications of Achilles Tendon Reconstruction", World journal of plastic surgery, 1 May 2016 (2016-05-01), pages 124-132, XP55729539, Iran Retrieved from the Internet: URL:https://www.ncbi.nlm.nih.gov/pmc/artic les/PMC5003947/pdf/wjps-5-124.pdf

## Description

### FIELD OF THE INVENTION

The present invention relates to a method for the preparation of a gel-forming composition for parenteral administration. The present invention pertains to the technical field of medical and veterinary sciences.

### BACKGROUND

Musculoskeletal diseases or disorders can affect the bones, muscles, joints, cartilage, tendons, ligaments, and other connective tissues that support and bind tissues and organs together. These diseases can develop over time or can result for instance by excessive use of the musculoskeletal system or from trauma. Musculoskeletal diseases are difficult to diagnose and/or treat due to the close relation of the musculoskeletal system to other internal systems.

Further and/or improved pharmaceutical formulations are configured for localized administration for the treatment of the musculoskeletal systems. Particular formulations of interest display or attain a gel consistency upon administration. These formulations often include pharmaceutical active ingredients and/or therapeutic cells.

EP 2 900 247 discloses a pharmaceutical formulation comprising solvent/detergent-treated plasma (S/D plasma) and hyaluronic acid.

EP 2 185 163 discloses a method for the treatment of a joint, said method comprises the infiltration of a compound in the joint, wherein said compound comprises at least one blood-derived substance.

WO 2013 076 160 discloses a gel-forming formulation comprising a glycosaminoglycan, a sustained release agent and one or more pharmaceutical active ingredients.

US2012171179 describes pharmaceutical compositions for the prevention of bone and cartilage diseases. The composition comprises adipose-derived stem cells, PRP, HA and calcium chloride.

WO2014/035721 is directed to modified plasma to be used as a hydrogel in wound healing and other applications.

WO2009/016451 is directed to methods for treating articular diseases or pain, based on joint infiltration with blood-derived substances such as PRP and plasma rich in growth factors. HA may be added to the composition.

Although the gel-forming compositions of the prior art have improved properties, designed for the treatment of the musculoskeletal system, the jellification properties of the gel-forming compositions is variable. These properties are often affected by the different treatment procedures within the preparation of the composition. Because of the intended mode of administration a reproducible gel-forming composition with controlled jellification properties should be at hand.

Thus there remains a need in the art for a method for the preparation of the gel-forming composition and an improved gel-forming composition with a controlled jellification. The present invention aims to resolve the problems and disadvantages mentioned above.

### SUMMARY OF THE INVENTION

The present invention and embodiments thereof serve to provide a solution to one or more of above-mentioned disadvantages. To this end, the present invention relates to a method for the preparation of a gel-forming composition for parenteral administration according to claim 1.

The method disclosed in current invention enables the preparation of a gel-forming composition, allowing a gradual and controlled jellification of the composition.

Preferred embodiments of the method are shown in any of the claims 2 to 13.

In a second aspect, the present invention relates to a composition according to claim 14. More particular, the composition as described herein provides an optimal jellification process so the composition can be safely handled and administered, and the composition jellifies at the site of injury establishing a cell-supportive environment and a sustained release of the pharmaceutical active ingredients comprised within the composition.

In a third aspect, the present invention relates to the composition for use according to claim 15. Preferred embodiments of this use are shown in claim 16-18.

### DEFINITIONS

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.
"Comprise", "comprising", and "comprises" as used herein are synonymous with "include", "including", "includes" or "contain", "containing" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "wt.%", here and throughout the description unless otherwise defined, refers to the relative weight of the respective component based on the overall weight of the formulation.

Whereas the terms "one or more", such as one or more member(s) of a group of members, is clear *per se,* by means of further exemplification, the term encompasses *inter alia* a reference to any one of said members, or to any two or more of said members, such as, *e.g.,* any ≥3, ≥4, ≥5, ≥6 or ≥7 etc. of said members, and up to all said members.

The terms "formulation", "composition", or "preparation" may be used interchangeably herein.

The term "plasma" is to be understood as a fraction obtained from a sample of whole blood, provided or contacted with an anticoagulant, e.g., CPDA-1 (comprising citrate, phosphate, dextrose, and/or adenosine) heparin, citrate, oxalate or EDTA. Cellular components of the blood sample, i.e. white and red blood cells are separated from the liquid component, i.e. plasma, by an appropriate technique e.g., centrifugation or apheresis. Afterwards, the plasma may optionally be solvent/detergent treated, e.g. to eliminate viruses. Solvent/detergent treatment enables the elimination of cells and/or cells debris e.g., lipid membranes, lipids and/or phospholipids. This is typically referred to as solvent/detergent treated plasma (S/D plasma). Plasma that did not undergo the solvent/detergent treatment can be labeled as non-treated plasma or native plasma. In an embodiment of the current invention, the plasma is not treated and without wishing to be bound to a theory, said plasma is understood to comprise cells, lipids and/or phospholipids. As a consequence, said plasma has similar or the same compositional characteristics with the native plasma. The "plasma" of current invention is preferably mammalian derived.

The term "fresh frozen plasma" is to be understood as a plasma frozen within twelve hours at or below -18°C following its preparation as referred in the plasma definition.

The term "apheresed plasma" is to be understood as a plasma obtained by plasmapheresis, a medical technology in which the blood is collected from the donor, passed through an apparatus (i.e. an apheresis) that separated the plasma from the all blood and returns the remainder fluid directly to the donor. Plasma that did undergo apheresis are contacted with anti-coagulant such as CPDA 1, ACD, heparin, citrate, oxalate or EDTA.

The term "centrifuged plasma" is to be understood as a plasma obtained by centrifugation of the all blood contacted with an anti-coagulant allowing the separation of the plasma from the all blood. Plasma that did undergo centrifugation can be contacted with anti-coagulant such as CPDA 1, ACD, heparin, citrate, oxalate or EDTA.

The term "gel-forming agent" as intended throughout this specification encompasses agents capable of forming, a solid, jelly-like material (gel). In particular, gel-forming agents form a gel when combined with or exposed to materials and/or conditions conducive to gel formation, for example but without limitation, when dissolved or dispersed in a suitable liquid phase, such as in an aqueous solution.

The term "hyaluronic acid" may be used interchangeably with "hyaluronate". The term "hyaluronic acid" refers to an anionic, non-sulfated polymer of disaccharides composed of D-glucuronic acid and N-acetyl-D-glucosamine, linked via alternating β-1,4 and β-1,3 glycosidic bonds. Hyaluronic acid derivatives include but are not limited to salts of hyaluronate such as sodium hyaluronate or an ester of hyaluronic acid with an alcohol of the aliphatic, heterocyclic or cycloaliphatic series, or a sulphated form of hyaluronic acid or combination of agents containing hyaluronic acid.

The term "subject" or "patient" are used interchangeably and refer to animals, preferably warm-blooded animals, more preferably vertebrates, even more preferably mammals. Preferred patients are equine, bovine, porcine, ovine, canine, feline subjects.

The term "a subject in need of treatment" includes subjects that would benefit from treatment of a given condition, particularly of a musculoskeletal disease such as a bone disease or a joint disease. Alternatively or in addition, said disease may affect tendons and/or ligaments. Such subjects may include, without limitation, those that have been diagnosed with said condition, those prone to develop said condition and/or those in whom said condition is to be prevented.

The terms "treatment" encompasses both the therapeutic treatment of an already developed disease or condition, as well as prophylactic or preventative measures, wherein the aim is to prevent or lessen the chances of incidence of an undesired affliction, such as to prevent the chances of progression of the disease or condition. Beneficial or desired clinical results may include, without limitation, alleviation of one or more symptoms or one or more biological markers, diminishment of extent of disease, stabilized state of disease, delay or slowing of disease progression, amelioration or palliation of the disease state, and the like. The term "treatment" can also mean prolonging survival as compared with expected survival if not receiving treatment.

The terms "sustained release" as used herein, broadly refer to the release of a compound from a composition over an extended, prolonged or increased period of time compared with the release of said compound from a reference composition such as a composition known in the prior art. As used herein, the sustained release refers to the prolonged release of one or more of the compounds of the composition, namely of the mammalian plasma or of one or more additional active pharmaceutical ingredient(s). For instance, it is known from the prior art that the half-life of high molecular weight hyaluronic acid in the knee joint is about 10 to 15 hours. The sustained release, as used herein, thus refers to the extended release of a hyaluronic acid from the present composition, for example release during one or more days, such as during 2 days, 3 days, 4 days, 5 days, 6 days, or during one or more weeks such as during 1.5 week, 2 weeks, 3 weeks, or during one or more months. These terms may thus also specifically encompass extended release, delayed release or controlled release.

The term "pharmaceutical compound" broadly refers to a compound, substance or component which, when provided in an effective amount, achieves a desired therapeutic and/or prophylactic outcome(s). Typically, an active pharmaceutical ingredient may achieve such outcome(s) through interacting with and/or modulating living cells or organisms. The term "pharmaceutical compound" also encompasses any pharmacologically active salts, esters, N-oxides or prodrugs of the title compound or substance.

The term "local administration" as used herein refers to a parenteral administration in or in the vicinity of a targeted site within the body.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

### DETAILED DESCRIPTION OF THE INVENTION

The present invention concerns a method for the preparation of a gel-forming composition. The preparation of a gel-forming composition is in general a delicate process wherein quality, quantity and activity of each of the compounds within the composition is crucial. The method of current invention is advantageous as the jellification of the gel-forming composition is controlled, occurs in a stable manner and allows parenteral administration.

In a first aspect, a method for the preparation of a gel-forming composition for parenteral administration is provided, said method comprises the steps of admixing a mixture of mammalian derived plasma comprising lipids and/or phospholipids and hyaluronic acid or a salt or ester thereof with a calcium solution, wherein the calcium concentration in the resulting gel-forming composition is between 0.2 and 1.4 mg/ml and the concentration of hyaluronic acid or a salt or ester thereof in said gel-forming composition is between 0.10 and 200 mg/ml.

In an embodiment, the mixture of mammalian derived plasma and hyaluronic acid or a salt or ester thereof is a pre-made mixture. In another embodiment, the mixture of mammalian derived plasma and hyaluronic acid or a salt or ester thereof can be mixed prior to the step of admixing said calcium solution. The calcium solution is added to said mixture. It was found that the sequence of steps aid in the uniform and stable jellification of said gel-forming composition. A gradual and controlled jellification is an absolute necessity, considering that said composition has as a purpose to be parenterally administered. Furthermore, the total concentration of said calcium in the gel-forming composition should be between 0.2 and 1.4 mg/ml in order to obtain the desired effect.

In an embodiment of the gel-forming composition, said composition comprises about 50 to 99 wt.% plasma, more preferably about 60 to 99 wt.%, more preferably about 70 to 99 wt.%, more preferably about 80 to 99 wt.%, more preferably about 90 to 99 wt.%, more preferably about 90, 91, 92, 93, 94, 95, 96, 97, 98, 99 wt.% plasma.

In an embodiment, a main component of the composition is mammalian derived plasma.

In a preferred embodiment of the invention, said plasma is mammalian derived, more preferably derived from equine, bovine, porcine, ovine, canine, or feline blood.

Preferably, the plasma may be mammalian plasma, such that the gel-forming composition comprising mammalian plasma is particularly suited for administration to mammalian subjects. Preferably the plasma is allogeneic. The collection process of allogeneic plasma is well-established and highly standardized with regard to the use of anticoagulant, separation and processing techniques, centrifugal force, and temperature and time, resulting in highly predictable amount of cells and solid components. Preferably the plasma is allogeneic equine, bovine, porcine, ovine, canine, or feline plasma.

In an embodiment, the plasma may include centrifuged or apheresed plasma. The mammalian plasma may be fresh frozen plasma. Preferably the mammalian plasma of current invention is apheresed fresh frozen plasma.

In an embodiment, said plasma is a non-solvent/detergent treated plasma.

Mammalian derived plasma includes plasma collected from single, or multiple donors from mammalian sources, such as equine, bovine, porcine, ovine, canine, or feline in plastic bags, or glass bottles and separated from blood cells via the means described above. After collection, mammalian derived plasma is stored at or below -18°C, preferably below -18°C.

Plasma comprises lipids and/or phospholipids, which may include, but are not limited to one or more sphingolipids, phosphatidylserines, phosphatidylcholines, phosphatidylethanolamines, cholesterol, cholesterol esters, triglycerides, diacylglycerides, lecithin, liposomes, minor plasma lipids, and combinations thereof. Plasma lipids and/or phospholipids promote complex formation with blood coagulation factors triggering the blood coagulation cascade.

The lipids and/or phospholipids may be externally added to the composition. In the current invention, said mammalian derived plasma will also be a source of lipids and/or phospholipids.

Various analytical methods are available for the determination of plasma lipid and/or phospholipid concentrations and characteristics. Chromatography is an analytical procedure for separating and analyzing lipids. Thin layer chromatography (TLC), gas chromatography (GC), and high-pressure liquid chromatography (HPLC) are often used for the assessment of plasma lipids and/or phospholipids. Also spectrometry is a known technique to assess the plasma lipids and/or phospholipids in plasma, like mass spectrometry or nuclear magnetic resonance (NMR) spectroscopy. Enzyme-linked immunosorbent assay (ELISA) is also a technique to assess plasma lipids and/or phospholipids in plasma.

The plasma lipids and/or phospholipids are preferably quantified by using HPLC technique. Limits are set on the amount of plasma lipids within the plasma of current invention enabling quality reproducibility of the plasma.

In the current invention, said plasma comprises lipids and/or phospholipids. Preferably the plasma comprises lipids and/or phospholipids, wherein said total concentration of lipids and/or phospholipids is between 0.2 and 7 mg/ml. More preferably the total concentration of lipids and/or phospholipids in the plasma is between 0.4 and 6 mg/ml, more preferably between 0.5 and 5 mg/ml. Without wishing to be bound to a theory said lipids and/or phospholipids are understood to have pro-coagulant activities in plasma and allow at least partially an improved jellification of the gel-forming composition.

In a further embodiment, said plasma comprises minor plasma lipids. Without wishing to be bound to a theory said minor plasma lipids are understood to have pro-coagulant activities in plasma and allow at least partially an improved jellification of the gel-forming composition.

In a preferred embodiment of the invention, said minor plasma lipids are preferably chosen from the group of palmitoyl ethanolamide (PEA), stearoyl ethanolamide (SEA), arachidonoyl ethanolamide (AEA).

In an embodiment, the total concentration of minor plasma lipids in the mammalian plasma is between 5 and 50 nmol/l, more preferably between 6 and 30 nmol/l.

In a more preferred embodiment of the invention, said composition comprises PEA, SEA and AEA, wherein each lipid is present at a concentration of between 0.2 and 14 nmol/l. In a further embodiment, each minor plasma lipid is present at a concentration between 0.5 and 12 nmol/l, more preferably between 0.8 and 8.5 nmol/l.

The jellification activity of plasma can be measured by assessing the coagulation parameters, preferably PTT and aPTT, and/or coagulation factors, preferable factor VIII and fibrinogen.

In an embodiment, the plasma used has an anti-hemophilic factor (factor VIII) activity higher than 75 %. Assays to quantify factor VIII activity are known in the art and may include clot-based assays, chromogenic assays or immunoassays such as ELISA. In an embodiment, said anti-hemophilic factor (factor VIII) activity is measured by a clot detection method using a viscosity-based detection system such as the STart^{®} semi-automated benchtop analyzer or the automated STA R Max^{®} 2, both from Stago (Cupaiolo R, Govaerts D, Blauwaert M, Cauchie P. Performance evaluation of a new Stago® automated haemostasis analyser: The STA R Max® 2. Int J Lab Hematol. 2019;41(6):731-737. doi:10.1111/ijlh.13100). In short, said method uses the increase in viscosity of the plasma being tested for clot detection. The change in viscosity is measured by monitoring the amplitude of an oscillating steel ball in a specially designed cuvette. Movement of the steel ball is mediated by two activating coils, working alternatively to induce and maintain a natural oscillation. When the start reagent is added, the detection and the chronometer start immediately and simultaneously. As the ball oscillates left and right, the amplitude of the motion is measured. The chronometer times the clotting of the sample. Amplitude is monitored during the entire clotting process. The amplitude remains constant while no clot is present. As the clot develops, the viscosity increases and the amplitude decreases. An algorithm is used to determine the clotting time.

In an embodiment, the plasma used has a fibrinogen (factor I) concentration ranging between 0.25 and 3 g/l. In an embodiment, the plasma used has an activated partial thromboplastin time (aPTT) activity ranging between 12 and 65 sec and/or a partial thromboplastin time (PTT) activity ranging between 5 and 17 sec. Suited method to measure (a)PTT is by means of a clot detection method using a viscosity-based detection system such as the STart^{®} semi-automated benchtop analyzer of Stago, which is described above. In a further preferred embodiment, said plasma complies with all of the above features. Assays to quantify factor VIII, fibrinogen concentration, aPTT activity, and PTT activity are known by a skilled person.

Fibrinogen circulates as a soluble plasma glycoprotein in the plasma. Fibrinogen is involved in the formation of fibrin and promotes blood clotting by forming bridges and activating blood platelets through binding.

Preferably the mammalian plasma of current invention has a total concentration of fibrinogen of between 0.25 and 3 g/l, more preferably of between 0.3 and 2.5 g/l, more preferably of between 0.4 and 2.1 g/l. Prior art documents prefer a defibrinated plasma, current invention however requires mammalian plasma with a minimal concentration of fibrinogen of 0.25 g/l.

The total protein concentration of the mammalian plasma of current invention is preferably between 10 and 100 g/l, preferably between 15 and 85 g/l, more preferably between 20 and 80 g/l. Without wanting to be bound to a theory, it is believed that protein levels above this concentration may lead to problems with clogging when administered to a subject.

In a more preferred embodiment said plasma has a concentration of white blood cells (WBCs) of less than 200 mil/l, preferably a concentration of WBCs of between 3 and 145 mil/l, more preferably between 5 and 85 mil/l, desirably between 10 and 50 mil/l.

The mammalian derived plasma comprises white blood cells, also called leukocytes, in an amount that does not interfere with the jellification of the composition.

In a particularly preferred embodiment said plasma has a concentration of blood platelets (BPs) of less than 50,000 mil/l, preferably a concentration of BPs of between 1,000 and 35,000 mil/l, more preferably a concentration of BPs of between 2,000 and 20,000 mil/l.

Blood platelets, also called thrombocytes, help in the blood coagulation process. Furthermore, platelets contain hundreds of proteins, known as growth factors. These platelet-derived growth factors are useful in regenerative medicine, in particular for stimulating repair of bone, cartilage, tendon or ligaments defects or for replacing damaged bones, cartilages, tendons or ligaments. The platelet concentration is lower than in whole blood. Although other documents in the prior art prefer the use of platelet-rich plasma to enhance wound healing, the concentration of platelets is specifically lower.

The method of the current invention comprises the step of admixing a mixture of mammalian derived plasma and hyaluronic acid (a glycosaminoglycan) or a salt or ester thereof with a calcium solution.

In an embodiment, the composition comprises one or more additional glycosaminoglycans. In certain embodiments, the additional glycosaminoglycan may be selected from the group consisting of a proteoglycan and derivatives thereof, a chondroitin sulfate, a keratan sulfate, a chitosan and derivatives thereof, a chitin and derivatives thereof; or mixtures of the aforementioned.

Hyaluronic acid plays an important role in the biological organism, firstly as a mechanical support of the cells of many tissues, such as the skin, the tendons, the muscles and cartilage and it is therefore the main component of the extracellular matrix. But hyaluronic acid also performs other functions in the biological processes, such as the hydration of tissues, lubrication of movable parts, cellular migration, cell function and differentiation.

In the current composition, the presence of hyaluronic acid will aid in biological processes such as tissue hydration, proteoglycan organization, cell differentiation, cell proliferation and angiogenesis. Hyaluronic acid derivatives maintain all the properties of said glycosaminoglycan, with the advantage of being able to be processed in various forms and having solubility and degradation times which, vary according to the type and percentage of derivation. Derivatives of hyaluronic acid may be a salt of hyaluronic acid, an ester of hyaluronic acid with an alcohol of the aliphatic, heterocyclic or cycloaliphatic series, or a sulphated form of hyaluronic acid. Without limitation, suitable derivatives may be salts of hyaluronic acid, such as preferably sodium hyaluronate.

In a preferred embodiment of current invention, said hyaluronic acid or a salt or ester thereof has a molecular weight less than 1,800 Kg/mol (1,800 kDa), more preferably between 700 and 1,600 Kg/mol (700 and 1,600 kDa), most preferably 700 and 1,000 Kg/mol (700 and 1,000 kDa). Preferably the hyaluronic acid or a salt or ester thereof has a high molecular weight. The high molecular weight of hyaluronic acid or a salt or ester should be understood as a molecular weight of between 700 and 1,000 Kg/mol (700 and 1,000 kDa). For example, injecting hyaluronic acid of a high molecular weight is effective for restoring the mechanical integrity of joints affected by osteoarthritis. Particularly preferred, the hyaluronic acid or salt or ester thereof has a narrow size distribution. It was observed that the molecular weight ranges as disclosed in embodiment ensures a suitable affinity for hyaluronic acid receptors and a better stimulation of the hyaluronic acid biosynthesis. Desirably, the hyaluronic acid or a salt or ester thereof has a high molecular weight and a narrow size distribution. Furthermore, hyaluronic acid or a salt or ester thereof with a molecular weight of between 700 and 1,000 Kg/ mol (700 and 1,000 kDa) improves the cell viability of the surrounding cells once implanted or injected in the tissue in need of a treatment. Furthermore, it was found that the hyaluronic acid of between 700 and 1,000 Kg/mol (700 and 1,000 kDa) enabled the quality and reproducibility of prepared product batches.

High molecular weight hyaluronic acid or a salt or ester thereof presents greater viscoelastic properties than the ones with a low molecular weight. The hyaluronic acid or a salt or ester thereof with a high molecular weight according to current embodiment admixed with the plasma of current invention, present even greater viscoelastic properties due to the rather native composition of the plasma. These great viscoelastic properties are of considerable interest, especially in view of the necessity to absorb major variations in pressure such as those which occur in vivo, like in joints.

Preferably the hyaluronic acid or a salt or ester thereof is an unbranched straight-chain hyaluronic acid.

Hyaluronic acid occurs naturally and does not have a species or organ specificity, even when implanted or injected into a living body. Hyaluronic acid shows excellent biocompatibility. Hence, the hyaluronic acid could be animal derived or of biofermentative origin. A well-known source of hyaluronic acid in the art is a rooster comb. Preferably the hyaluronic acid is of biofermentative origin.

In the current invention, the gel-forming composition comprises the hyaluronic acid or a salt or ester thereof in a concentration ranging from about 0.10 to 200 mg/ml, preferably from about 1 to 100 mg/ml, more preferably from about 2 to 50 mg/ml, more preferably from about 4 to 20 mg/ml.

In an embodiment, the hyaluronic acid or a salt or ester thereof is preferably sterilized. Hyaluronic acid can be steam sterilized or filter sterilized. Preferably the hyaluronic acid is filter sterilized, as steam sterilization may affect molecular weight distribution range of the hyaluronic acid fibers. The hyaluronic acid is preferably solubilized and sterilized by filtration.

Preferably the mammalian plasma is sterilized through a sterile filter.

The filter sterilization of the mammalian plasma and hyaluronic acid has beneficial influences on the quality and reproducibility of the preparation of the gel-forming composition.

The mixture of at least plasma according to one of the embodiments as described above and hyaluronic acid is made. The mammalian derived plasma and hyaluronic acid or a salt or ester thereof are admixed, resulting in a plasma and hyaluronic acid mixture.

In an embodiment, said mixture is freeze-dried. As a consequence, a lyophilized product is obtained. Freeze-drying may also be known as lyophilization in the art. The following terms "lyophilized product" and "freeze-dried mixture" are used interchangeably herein.

In a preferred embodiment, the plasma and hyaluronic mixture is diluted prior to freeze-drying. Preferably said mixture is solubilized at least 4 times, preferably at least 3 times, more preferably at least 2 times, desirably 2 times in aqueous solution to reduce the product viscosity and facilitate further manufacturing steps.

In another embodiment said plasma and hyaluronic acid mixture is freeze-dried prior to the addition of said calcium solution.

The freeze-drying of said mixture increases the shelf-life. Freeze-drying of said mixture provides a lyophilized and stable product for months. The lyophilized product can be easily stored, shipped and reconstituted for injection. The lyophilized product is stable at a temperature of between 15 and 25°C. Having a lyophilized product with a stability at room temperature is beneficial for the medical practitioner. The product can be easily transported or handled, without substantially deteriorating the quality of the product. There is no need for special a cooling container.

In a preferred embodiment, the calcium solution is added after the plasma and hyaluronic acid mixture is freeze-dried. The calcium solution is preferably added after the plasma and hyaluronic acid mixture is freeze-dried, and after possibly supplementing the mixture with one or more pharmaceutical compounds.

Subsequently, once the mixture of plasma and hyaluronic acid is made and freeze-dried to obtain said lyophilized product, the calcium solution is added, such that the final calcium concentration in the gel-forming composition is between 0.2 and 1.4 mg/ml. Preferably the total concentration of said calcium in said composition is between 0.4 and 1.2 mg/ml, more preferably between 0.6 and 1.0 mg/ml, more preferably 0.65 and 0.95 mg/ml, more preferably 0.7 and 0.9 mg/ml, more preferably 0.75 and 0.85 mg/ml.

The calcium solution of current invention makes jellification of the gel-forming composition possible. Said calcium source may be selected from a suitable amount of pharmaceutically acceptable calcium salt(s), preferably soluble calcium salt(s). Such Ca²⁺salts may be formed with inorganic or organic acids. Examples of such salts include calcium chloride (CaCl₂), calcium glycerophosphate, calcium phosphate, calcium hydrogen carbonate, calcium sulphate, calcium lactate, calcium gluconate, calcium ascorbate, and mixtures thereof. Particularly preferred is calcium chloride, which displays advantageously good solubility and is well-tolerated in injectable solutions.

In the current invention, the calcium source is a calcium solution. Preferably, the calcium solution is prepared by adding a source of calcium, preferably calcium chloride to an acidic solution, such as for instance a hydrogen chloride solution. Preferably the final pH of the acidic solution in the composition is between 1 and 4, more preferably between 1.5 and 2.5.

Said acidic solutions are preferably pharmaceutically acceptable acids. Pharmaceutically acceptable acids may include, but are not limited to (i) an inorganic acid such as hydrochloric acid, hydrobromic acid, hydroiodic acid and the like, (ii) an organic mono-, di- or tri-carboxylic acid (for example, formic acid, acetic acid, adipic acid, alginic acid, citric acid, ascorbic acid, aspartic acid, benzoic acid, butyric acid, camphoric acid, gluconic acid, glucuronic acid, galacturonic acid, glutamic acid, heptanoic acid, hexanoic acid, fumaric acid, lactic acid, lactobionic acid, malonic acid, maleic acid, nicotinic acid, oxalic acid, pamoic acid, pectinic acid, 3-phenylpropionic acid, picric acid, pivalic acid, propionic acid, succinic acid, tartaric acid, undecanoic acid and the like) or (iii) a sulfonic acid (for example, benzenesulfonic acid, sodium bisulfate, sulfuric acid, camphorsulfonic acid, dodecylsulfonic acid, ethanesulfonic acid, methanesulfonic acid, isethionic acid, naphthalenesulfonic acid, p-toluenesulfonic acid and the like).

In an embodiment of the invention said calcium solution is a calcium chloride solution. In a particularly preferred embodiment of the invention, said calcium chloride solution has an acidic pH preferably between a pH of 1 and 4. Said calcium chloride solution is preferably admixed with the freeze-dried mixture to dissolve the mixture prior to administration. Said calcium chloride solution enables the formation of a gel with high cohesive properties, allowing an optimal integration of the gel-forming composition at the site of injury. Once admixed, the pH of the composition is physiologic, preferably between a pH of 5 and 8, more preferably between a pH of 6 and 7.8

The gel-forming composition may comprise one or more pharmaceutical compounds in addition to the mammalian plasma and hyaluronic acid or a salt or ester thereof. In other embodiments, the plasma and glycosaminoglycan may be the only compounds of the composition; hence, in such embodiments the composition may consist of or consist essentially of the plasma and glycosaminoglycan.

Depending on the nature of the pharmaceutical compound, said compound may be added at different timing within the preparation of the gel-forming composition. In an embodiment, said pharmaceutical compounds are mixed with the plasma and hyaluronic acid mixture. In an embodiment, said pharmaceutical compounds are mixed with the plasma and hyaluronic acid mixture prior to freeze-drying said mixture. In an embodiment, said pharmaceutical compounds are mixed with the freeze-dried plasma and hyaluronic acid mixture. In a preferred embodiment, said pharmaceutical compounds are mixed with the plasma and hyaluronic acid mixture, freeze-dried or not, prior to the addition of the calcium solution.

In certain embodiments, the composition may advantageously further comprise one or more pharmaceutical compounds. The applicability of the present invention is not limited to any pharmaceutical compound or class of pharmaceutical compound. The pharmaceutical compound may be pharmacologically active itself, or may be converted into a pharmacologically active species by a chemical or enzymatic process in the body, i.e., the pharmaceutical compound may be a prodrug. The current gel-forming composition may be particularly useful for poorly-stable pharmaceutical compounds. Illustrative non-limiting examples of poorly-stable pharmaceutical compounds include peptides and proteins such as growth factors, peptide-like active ingredients, antibodies and vaccines, small interfering RNA (siRNA), DNA, hormones, etc.

Moreover, combination of two or more pharmaceutical compounds or dose combinations may be included as the drug component. In this case, the release of each compound may be identical or different such as for instance in case of a combination of two compounds in which the first one is presented as an immediate release form and the second one as a controlled release. Similarly, a combination of immediate release and controlled release form may also be obtained for the same compound, in order to provide a rapid and sustained effect.

In an embodiment of current invention said gel-forming composition further comprises one or more pharmaceutical compounds, wherein said compounds are selected from the group consisting of an active pharmaceutical ingredient, an antibiotic agent, a cell composition, a small organic molecule, a protein or a peptide.

In a preferred embodiment said active pharmaceutical ingredient is an alpha-2 adrenergic receptor agonist, preferably clonidine.

In a specific embodiment the alpha-2 adrenergic receptor agonist may be selected from the group consisting of clonidine and derivatives thereof, including 2,6-dimethylclonidine, 4-azidoclonidine, 4-carboxyclonidine-methyl 3,5-dichlorotyrosine, 4-hydroxyclonidine, 4-iodoclonidine, alinidine, apraclonidine, chlorethylclonidine, clonidine 4-isothiocyanate, clonidine 4-methylisothiocyanate, clonidine receptor, clonidine-displacing substance, hydroxyphenacetyl aminoclonidine, N,N'-dimethylclonidine, p-aminoclonidine, and tiamenidine; imidazolidines, including imidazolines, impromidine, detomidine, medetomidine, dexmedetomidine, levamisole, losartane, lofexidine, miconazole, naphazoline, niridazole, nitroimidazoles, ondansetron, oxymetazoline, phentolamine, tetramisole, thiamazole, tizanidine, tolazoline, trimetaphan; imidazoles, including 4-(3-butoxy-4-methoxybenzyl) imidazolidin-2-one, urocanic acid, amino-imidazole carboxamide, antazoline, biotine, bis (4-methyl-1-homo piperazinylthiocarbonyl) disulfide, carbimazole, cimetidine, clotrimazole, creatinine, dacarbazine, dexmedetomidine, econazole, enoximone, ethymizol, etomidate, fadrozole, fluspirilene, idazoxan, mivazerol; guanidines, including agmatine, betanidine, biguanides, cimetidine, creatine, gabexate, guanethidine, guanethidine sulfate, guanclofine, guanfacine, guanidine, guanoxabenz, impromidine, iodo-3 benzylguanidine, methylguanidine, mitoguazone, nitrosoguanidines, pinacidil, robenidine, sulfaguanidine, zanamivir; alpha-methyinorepherine, azepexole, 5-bromo-6-(2 imidazolidine-2-ylamino) quinoxalin, formoterol fumarate, indoramin, 6-allyl-2-amino-5,6,7,8-tetrahydro4H-thiazolo [4,5-d]azepine diHCl, nicergoline, rilmenidine, N-(2-bromo-6-fluorophenyl)-4,5-dihydro-1H-imidazol-2-amine and xylazine.

Clonidine is a centrally acting alpha-2 adrenergic receptor agonist. It is generally used as an anti-hypertensive agent. Clonidine is also commonly referred to as 2,6-dichloro-N-2-imidazolidinyldenebenzenamine (C₉H₉Cl₂N₃). The concentration of clonidine in the gel-forming composition is preferably between 0.01 and 0.5 mg/ml, more preferably between 0.03 and 0.3 mg/ml, more preferably between 0.05 and 0.2 mg/ml. Said low concentrations of clonidine or a derivative thereof in the gel-forming composition is effective in the treated tissue. These low concentrations of clonidine are effective in the injured tissue, as the gel forms an ideal matrix for clonidine to remain active and to be slowly released into the injured tissue.

In a preferred embodiment said cell composition comprises one or more antibiotic agents. In the context of the present invention, an antibiotic agent is a substance that has the capacity to inhibit the growth of or to destroy bacteria and other microorganisms. In a more preferred embodiment the antibiotic agent is selected from the classes consisting of beta-lactam antibiotics, aminoglycosides, ansa-type antibiotics, anthraquinones, antibiotic azoles, antibiotic glycopeptides, macrolides, antibiotic nucleosides, antibiotic peptides, antibiotic polyenes, antibiotic polyethers, quinolones, antibiotic steroids, sulfonamides, tetracycline, dicarboxylic acids, antibiotic metals, oxidizing agents, substances that release free radicals and/or active oxygen, cationic antimicrobial agents, quaternary ammonium compounds, biguanides, triguanides, bisbiguanides and analogs and polymers thereof and naturally occurring antibiotic compounds. Preferably the antibiotic agent is an aminoglycoside, preferably gentamicine.

In another or further embodiment said cell composition is comprises mesenchymal stem cells, osteoprogenitor cells, osteoblasts, osteocytes, chondroblasts and/or chondrocytes.

The mesenchymal stem cells (MSC) are capable of undergoing osteogenic or chondrogenic differentiation. Other cells are already committed towards osteoblastic or chondroblastic lineage. Preferably, the cells of the cell composition of current invention may be animal cells, preferably warm-blooded animal cells, more preferably mammalian cells. The mammalian cells are preferably of equine, bovine, porcine, ovine, canine, or feline origin.

Without limitation, the composition may comprise an amount of cells ranging from about 0.05×10⁶ to 5×10⁹ cells, preferably from about 0.5×10⁶ to 1×10⁹ cells, more preferably from about 4×10⁶ to 250×10⁶ cells.

In an embodiment, said cell composition is added after the lyophilization of the plasma and hyaluronic acid mixture in order to prevent disruption of said cells.

In another or further preferred embodiment said small organic molecule is a scaffold or matrix component with osteoconductive properties, preferably tricalcium phosphate particles (TCP).

The osteoconductive matrices further allow for a sustained release of the pharmaceutical compounds, like for instance clonidine, in the composition of current invention and promote bone repair. The matrix allows influx of the cells as discussed above facilitating bone formation and repair of the fracture site. Tricalcium phosphate particles are mineral materials effective to provide a scaffold or matrix for bone ingrowth. Preferably TCP have an average particle diameter between about 5 and 200 µm, more typically between about 10 and 100 µm, and desirably between about 20 and 60 µm. Methods to measure the particle size of TCP are known in the art and may include sedimentometry, analytical centrifugation and various kinds of spectrometry. In an embodiment, said TCP particle size is measured by laser diffraction. The concentration of TCP in the composition is preferably between 10 and 200 mg/ml, more preferably between 30 and 150 mg/ml, more preferably between 50 and 100 mg/ml. The osteoconductive matrices of current invention can take on any shape and are malleable, cohesive and can be injected at or near the target tissue site.

The gel-forming composition of current invention may additionally comprise proteins or (poly)peptides. Biologically active proteins, peptides and polypeptides suitable for administration in the composition of current invention include growth hormones, growth factors, and other biologically active fragments and derivatives thereof. Preferred proteins include equine, bovine, porcine, ovine, canine, or feline growth hormones; and is meant to encompass those which are of natural, synthetic, recombinant or biosynthetic origin. Additionally, metals or metal compounds associated with biologically active proteins, peptides and polypeptides, as well as acid salts, derivatives and complexes and anti-hydrating agents are suitable for incorporation into the composition of the invention.

The present composition may comprise, in addition to the herein particularly specified pharmaceutical compounds, one or more pharmaceutical excipients. Suitable excipients depend on the dosage form and identities of said compounds and can be selected by the skilled person. Pharmaceutical excipients should not interfere with the activity of the plasma, hyaluronic acid or pharmaceutical compounds.

In an embodiment, the composition may further comprise whole blood or a fractionated component of whole blood. The addition of whole blood or said fractionated component, preferably of whole blood, to the present composition may allow at least partially to improve the regenerative properties. The composition comprising whole blood or said fractionated component thereof advantageously comprise platelet-derived growth factors useful in regenerative medicine, in particular for stimulating repair of bone, cartilage, tendon or ligaments defects or for replacing damaged bones, cartilages, tendons or ligaments. For example, the whole blood may be allogeneic or autologous with respect to the subject receiving the formulation.

In an embodiment, the composition is devoid or substantially devoid of serum.

In another embodiment, the composition may further comprise one or more substance with osteogenic, osteo-inductive and/or osteo-conductive properties. In preferred embodiments, such substance may be selected from the group comprising or consisting of a fibroblast growth factor (FGF), preferably FGF-2, a transforming growth factor beta (TGFB), preferably TGFB-1, platelet-derived growth factor (PDGF), interleukin-8 (IL-8), a bone morphogenetic protein (BMP), for example any one or more of BMP-2, BMP-4, BMP-6 and BMP- 7, parathyroid hormone (PTH), parathyroid hormone-related protein (PTHrp), and stem cell factor (SCF). Hence, in certain embodiments, the pharmaceutical active protein or peptide may be a growth factor, preferably a growth factor selected from the group consisting of a FGF, a TGFB, PDGF, IL- 8, a BMP, PTH, PTHrp, and SCF, more preferably a growth factor selected from the group consisting of FGF-2, TGFB-1, PDGF, IL-8, BMP-2, BMP-4, BMP-6, BMP-7, PTH, PTHrp, and SCF.

In another embodiment said gel-forming composition is configured for parenteral administration, preferably for intraosseous, periosseous, intraarticular, periarticular administration, or for intratendon, peritendon, intraligament, or periligament administration.

The composition according to current invention is preferably configured for parenteral administration. For example, the composition may be in the form of a parenterally acceptable aqueous solution, which is pyrogen-free and has suitable pH, isotonicity and stability. Parenteral administration has the advantage that a local injection can take place and the site of injury can be specifically targeted and treated. Said targeted treatment by the composition of current invention safe and efficient and does not involve complex functionality measurements like medicines for oral administration. Depending on the site of injury different locations of application routes are favored. A person skilled in the art is familiar with these application routes. The gel-forming composition is preferably administered by injection, using a needle and a syringe.

In a particular embodiment the composition is configured for intraosseous or periosseous administration. Intra-osseous administration or delivery generally refers to a method whereby a treatment is delivered, directly or indirectly, into the bone (trabecular or cortical). Peri-osseous administration or delivery generally refers to a method whereby a treatment is delivered in the surroundings of a bone (especially around the fracture/damage site). In another particular embodiment, the composition is configured for intraarticular or periarticular administration. Intraarticular administration or delivery generally refers to a method whereby a treatment is delivered, directly or indirectly, into the synovial capsule of an articulating joint. Peri-articular administration or delivery generally refers to a method whereby a treatment is delivered in the surroundings of the synovial capsule of an articulating joint and/or the subchondral bone. In a further embodiment, the composition is configured for intratendon or peritendon administration. In another and further embodiment the composition is configured for intraligament or periligament administration.

A second aspect of current invention relates to a composition obtained by the method of current invention. Advantageously said composition will jellify within 30 minutes after the addition of said calcium solution.

The gel-forming composition changes from a liquid substance to a jelly substance within 30 minutes. This timeframe is for a practitioner enough to safely handle and administer the gel-forming composition to the site of injury. Preferably the composition jellifies within 25 minutes after the addition of said calcium solution, more preferably the composition jellifies within 20 minutes after said calcium solution is added. Thereby the gel-forming composition can still be handled by the practitioner while the composition is still liquid and the composition arrives at the site of injury in an almost jelly state, enabling a targeted delivery and sustained release of the biological substances incorporated in the composition.

Hence, in an embodiment of the current invention, the calcium solution is only added to the plasma and hyaluronic acid mixture just prior to administration of the final composition. As such, a controlled delivery of the composition can be obtained.

In an embodiment, the composition of current invention is for use in the treatment of musculoskeletal diseases, preferably wherein the musculoskeletal disease is a bone disease or a joint disease.

Alternatively or in addition, said musculoskeletal disease may affect tendons and/or ligaments. Also intended is the use of the gel-forming composition as described above for the manufacture of a medicament for the treatment of a musculoskeletal disease, preferably a bone disease or a joint disease. Alternatively or in addition, said musculoskeletal disease may affect tendons and/or ligaments. Further intended is a method for treating a musculoskeletal disease, preferably a bone disease or a joint disease (alternatively or in addition, said disease may affect tendons and/or ligaments), in a subject in need of such treatment, comprising administering to said subject a therapeutically or prophylactically effective amount of the gel-forming composition as described above.

In an embodiment, the current invention relates to a composition or kit for use in the treatment of osteoarthritis. Osteoarthritis is a progressively worsening inflammation of the joint caused by the deterioration of cartilage. In a healthy joint, cartilage acts as a cushion to allow the joint to move smoothly through its full range of motion. In cases of osteoarthritis, this cartilage cushion begins to break down because of factors such as age, injury, repetitive stress, disease or genetic predisposition. The loss of this protective cushion results in pain, inflammation, decreased range of motion, and the development of bone spurs. Diagnosis is typically based on signs and symptoms, with medical imaging and other tests used to support or rule out other problems.

In an embodiment, the current invention relates to a composition or kit for use in the prevention of cranial cruciate ligament (CCL) rupture. The cranial cruciate ligament is one of the most important stabilizers inside the knee joint, the middle joint in the back leg. Rupture of the cranial cruciate ligament is one of the most common reasons for hind limb lameness, pain, and subsequent knee arthritis. While the clinical signs associated with CCL rupture vary, the condition invariably causes rear limb dysfunction and pain. Trauma accounts for a minority of CCL ruptures, whereas progressive degeneration of the ligament has been attributed to a variety of factors that may be broadly classified as genetic, conformational, environmental, immune-mediated, and inflammatory. Diagnosing complete tears of the CCL is easily accomplished using a combination of gait observations, physical examination findings, and radiography (X-rays). By contrast, partial CCL tears may be more challenging to diagnose. Use of the current composition or kit for treating and/or preventing partial rupture of the cranial cruciate ligament in a subject such as an animal, significantly reduces signs of ligament degeneration and helps prevent complete cranial cruciate ligament rupture and reduces the incidence of contralateral disease in subjects with unilateral CCL rupture.

In an embodiment, the current invention relates to a composition or kit for use in the treatment of tendinopathies, such as supraspinatus tendinopathy and Achilles tendon rupture. Tendinopathy is a type of tendon disorder that results in pain, swelling, and impaired function. Supraspinatus tendinopathy is a term used to describe tears, calcifying tendinopathy, tendinosis and/or injuries in and around the tendon of the supraspinatus muscle, and is a cause of forelimb lameness. The supraspinatus is an important passive stabiliser of the shoulder joint, and is responsible for shoulder extension and advancing the limb. Injury to the tendon of the supraspinatus muscle causes inflammation. Tearing of the tendon fibers and the resulting inflammation can lead to mineralization and calcification of the tendon, which are a source of pain and lameness. The Achilles tendon's main function is rear-limb forward progression, and it contributes to passive support of the hock. The etiology of Achilles tendon injuries is usually traumatic. Depending on the trauma, the severity of the lesion may vary considerably, leading to stretching, small or partial lacerations or a complete rupture.

Accordingly, a related aspect provides the gel-forming composition comprising mammalian derived plasma and hyaluronic acid or a salt or ester thereof for use as an excipient, preferably as excipient in a pharmaceutical formulation configured for parenteral administration, more preferably for intra-osseous, peri-osseous, intraarticular, or peri-articular administration, or for intra-tendon, peri-tendon, intraligament or peri-ligament administration.

The gel-forming composition may be administered as a single therapeutically effective dose or administered as multiple therapeutically effective doses of the composition for parenteral administration, according to the patient's physiopathological condition and the protocol established by the medicinal practitioner.

In an embodiment, the composition according to the current invention is intraarticular or locally administered in a single or multiple therapeutically effective dose(s). The dose is adapted to the targeted joint and the body weight of the subject.

Specifically in the case of tendinopathies, the composition according to the current invention is periarticular, peritendinous or intratendinous administered in a single or multiple therapeutically effective dose(s). The dose is adapted to the targeted site and the body weight of the subject.

Also intended is the use of the composition as described above for the manufacture of a medicament for the treatment of a musculoskeletal disease, preferably a bone disease or a joint disease, more preferably osteoarthritis, prevention of CLL rupture or tendinopathies such as supraspinatus tendinopathy and Achilles tendon rupture.

The invention is further described by the following examples which further illustrate the invention.

### EXAMPLES

The present invention will now be further exemplified with reference to the following example(s).

### Example 1: Preparation protocol of the gel-forming composition

The gel-forming composition is prepared in different product batches. In addition to preparing a gel-forming composition with optimal properties, each product batch should show similar optimal properties.

The frozen canine plasma prepared by apheresis is thawed at 37°C for 60 minutes, preferably 40 minutes, more preferably 30 minutes. Said canine plasma is filtered through a filter with a filter size within the range of between 0.4 µm and 5 µm, more preferably 0.6 µm and 1.2 µm and then sterile filtered. The filtered canine plasma and hyaluronic acid are mixed to constitute the mixture. Said mixture is solubilized 4 times, preferably 3 times and more preferably 2 times in aqueous solution to reduce the product viscosity and facilitate further manufacturing steps. Following, said mixture is freeze-dried during a process lasting 72 to 120 hours. The duration of the process is optimized so that a more aerated lyophilizate product is presented, which later on can be easily reconstituted. In a next step, the calcium chloride powder is dissolved in a hydrogen chloride solution and the dissolved calcium chloride is sterile filtered. Here, the hydrogen chloride solution acts as a diluent the calcium chloride powder.

### Example 2: Formulating the ael-formina composition before use.

A vial comprising the freeze-dried canine plasma and hyaluronic acid lyophilized product is reconstituted with the sterilized calcium chloride solution. The time of resuspension counts for only 5 minutes. Other gel-forming composition have a resuspension time, i.e. longer than two hours. The said freeze-dried mixture may comprise clonidine in a concentration of between 0.05 and 0.2 mg/ml. This composition once resuspended can be parenterally administered.

### Example 3: Comparative examples of the gel-forming composition

The gel-forming composition preferably includes 99% non S/D treated plasma, 5-24 mg/ml sodium hyaluronate, 0.05-0.2 mg/ml clonidine, 0.6-1 mg/ml calcium chloride and 0.4-0.65 mg/ml HCl.

Changing the concentration of calcium chloride from 0.10 mg/ml to 2 mg/ml results in a fast jellification of the composition. Consequently, the practitioner cannot to properly administer the composition at the site of injury. On the other hand, a concentration of 0.05 mg/ml calcium chloride reveals a composition without an insufficient jellification, in that the composition remains liquid.

The concentration of hyaluronic acid or a salt or ester thereof too has an impact on the jellification of the gel-forming composition, which is shown by a composition with a low concentration of sodium hyaluronate (0.05 mg/ml) and a high concentration of sodium hyaluronate (250 mg/ml). Similar results were seen as for the calcium chloride.

Also the plasma has an influence of the jellification of the composition. Indeed, the presence of lipids and/or phospholipids in the non-treated plasma promotes the jellification process by triggering coagulation cascade. In case solvent-detergent plasma is used, an insufficient jellification takes places, due to the lack of plasma lipids and/or phospholipids.

The concentrations and properties of said plasma, hyaluronic acid or a salt or ester thereof and calcium chloride influence the jellification of the gel-forming composition and should therefore preferably fall within the disclosed ranges of current invention.

## Claims

1. A method for the preparation of a gel-forming composition for parenteral administration, said method comprises the steps of admixing a mixture of mammalian derived plasma comprising lipids and/or phospholipids and hyaluronic acid or a salt or ester thereof with a calcium solution, wherein the calcium concentration in the resulting gel-forming composition is between 0.2 and 1.4 mg/ml and the concentration of hyaluronic acid or a salt or ester thereof in said gel-forming composition is between 0.10 and 200 mg/ml.

2. The method according to claim 1, **characterized in that** said calcium solution is a calcium chloride solution.

3. The method according to claim 1 or 2, **characterized in that** said calcium chloride solution has an acidic pH.

4. The method according to any one of the preceding claims 1-3, **characterized in that** said plasma and hyaluronic acid mixture is freeze-dried prior to the addition of said calcium solution.

5. The method according to any one of the preceding claims 1-4, **characterized in that** said lipids and/or phospholipids are present at a total concentration of between 0.2 and 7 mg/l.

6. The method according to any one of the preceding claims 1-5, **characterized in that** said lipids comprises minor plasma lipids, wherein said minor plasma lipids are preferably chosen from the group of palmitoyl ethanolamide (PEA), stearoyl ethanolamide (SEA), arachidonoyl ethanolamide (AEA), and wherein a total concentration of minor plasma lipids is between 5 and 50 nmol/l.

7. The method according to any one of the preceding claims 1-6, **characterized in that** said plasma has a concentration of white blood cells (WBCs) of less than 200 mil/l, preferably a concentration of WBCs of between 3 and 145 mil/l, more preferably between 5 and 85 mil/l.

8. The method according to any one of the preceding claims 1-7, **characterized in that** said plasma has a concentration of blood platelets (BPs) of less than 50,000 mil/l, preferably a concentration of BPs of between 1,000 and 35,000 mil/l, more preferably a concentration of BPs of between 2,000 and 20,000 mil/l.

9. The method according to any one of the preceding claims 1-8, **characterized in that**
the hyaluronic acid or a salt or ester thereof has a molecular weight less than 1,800 Kg/ mol (1,800 kDa), more preferably between 700 and 1,600 Kg/mol (700 and 1,600 kDa), more preferably 700 and 1,000 Kg/mol (700 and 1,000 kDa).

10. The method according to any one of the preceding claims 1-9, **characterized in that** said plasma and hyaluronic acid mixture further comprise one or more pharmaceutical compounds chosen from the group of an active pharmaceutical ingredient, an antibiotic agent, a cell composition, a small organic molecule, a protein, and a peptide.

11. The method according to any of the preceding claims 1-10, **characterized in that** said active pharmaceutical ingredient is an alpha-2 adrenergic receptor agonist, preferably clonidine.

12. The method according to any one of the preceding claims 1-11, **characterized in that** said small organic molecule is a scaffold or matrix component with osteoconductive properties, preferably tricalcium phosphate particles (TCP).

13. The method according to any of the preceding claims 1-12, **characterized in that** said gel-forming formulation is configured for parenteral administration, preferably for intraosseous, periosseous, intraarticular, periarticular administration, or for intratendon, peritendon, intraligament, or periligament administration.

14. A composition obtained by the method according to any one of the preceding claims 1 to 13.

15. The composition according to claim 14 for use in the treatment of musculoskeletal diseases in a subject, wherein the musculoskeletal disease is preferably a bone disease or a joint disease.

16. The composition for use according to claim 15 in the treatment of osteoarthritis.

17. The composition for use according to claim 15 in the treatment or prevention of cranial cruciate ligament rupture.

18. The composition for use according to claim 15 in the treatment of tendinopathies.

## Patentansprüche

1. Ein Verfahren zur Herstellung einer gelbildenden Zusammensetzung zur parenteralen Verabreichung, wobei das Verfahren die Schritte des Vermischens einer Mischung aus einem von einem Säugetier stammenden Plasma, das Lipide und/oder Phospholipide und Hyaluronsäure oder ein Salz oder einen Ester davon umfasst, mit einer Calciumlösung, wobei die Calciumkonzentration in der entstandenen gelbildenden Zusammensetzung zwischen 0,2 und 1,4 mg/ml liegt und die Konzentration von Hyaluronsäure oder eines Salzes oder Esters davon in der gelbildenden Zusammensetzung zwischen 0,10 und 200 mg/ml liegt.

2. Das Verfahren nach Anspruch 1, **dadurch gekennzeichnet, dass** die Calciumlösung eine Calciumchloridlösung ist.

3. Das Verfahren nach Anspruch 1 oder 2, **dadurch gekennzeichnet, dass** die Calciumchloridlösung einen pH-Wert im sauren Bereich aufweist.

4. Das Verfahren nach einem der vorhergehenden Ansprüche 1 bis 3, **dadurch gekennzeichnet, dass** die Plasma- und Hyaluronsäuremischung vor dem Zusetzen der Calciumlösung gefriergetrocknet wird.

5. Das Verfahren nach einem der vorhergehenden Ansprüche 1 bis 4, **dadurch gekennzeichnet, dass** die Lipide und/oder Phospholipide in einer Gesamtkonzentration zwischen 0,2 und 7 mg/l vorhanden sind.

6. Das Verfahren nach einem der vorhergehenden Ansprüche 1 bis 5, **dadurch gekennzeichnet, dass** die Lipide kleine Plasmalipide sind, wobei die kleinen Plasmalipide vorzugsweise aus der Gruppe von Palmitoylethanolamid (PEA), Stearoylethanolamid (SEA), Arachidonoylethanolamid (AEA) ausgewählt werden und wobei eine Gesamtkonzentration kleiner Plasmalipide zwischen 5 und 50 nmol/l liegt.

7. Das Verfahren nach einem der vorhergehenden Ansprüche 1 bis 6, **dadurch gekennzeichnet, dass** das Plasma eine Konzentration weißer Blutkörperchen (WBCs) von weniger als 200 mil/l aufweist, vorzugsweise eine Konzentration von WBCs zwischen 3 und145 mil/l, bevorzugter zwischen 5 und 85 mil/l.

8. Das Verfahren nach einem der vorhergehenden Ansprüche 1 bis 7, **dadurch gekennzeichnet, dass** das Plasma eine Konzentration von Blutplättchen (PBs) von weniger als 50.000 mil/l, vorzugsweise eine Konzentration von BPs zwischen 1.000 und 35.000 mil/l, bevorzugter eine Konzentration von BPs zwischen 2.000 und 20.000 mil/l aufweist.

9. Das Verfahren nach einem der vorhergehenden Ansprüche 1 bis 8, **dadurch gekennzeichnet, dass** die Hyaluronsäure oder ein Salz oder Ester davon eine Molekülmasse von weniger als 1.800 kg/mol (1.800 kDa) aufweist, bevorzugter zwischen 700 und 1.600 kg/mol (700 und 1.600 kDa), bevorzugter zwischen 700 und 1.000 kg/mol (700 und 1.000 kDa).

10. Das Verfahren nach einem der vorhergehenden Ansprüche 1 bis 9, **dadurch gekennzeichnet, dass** die Plasma- und Hyaluronsäuremischung ferner eine oder mehrere pharmazeutische Verbindungen umfasst, die aus der Gruppe eines aktiven pharmazeutischen Bestandteils, eines antibiotischen Mittels, einer Zellzusammensetzung, eines kleinen organischen Moleküls, eines Proteins und eines Peptids ausgewählt wird.

11. Das Verfahren nach einem der vorhergehenden Ansprüche 1 bis 10, **dadurch gekennzeichnet, dass** der aktive pharmazeutische Bestandteil ein Alpha-2-Adrenozeptoragonist ist, vorzugsweise Clonidin.

12. Das Verfahren nach einem der vorhergehenden Ansprüche 1 bis 11, **dadurch gekennzeichnet, dass** das kleine organische Molekül eine Gerüst- oder Matrixkomponente mit osteokonduktiven Eigenschaften ist, vorzugsweise Tricalciumphosphatpartikel (TCP).

13. Das Verfahren nach einem der vorhergehenden Ansprüche 1 bis 12, **dadurch gekennzeichnet, dass** die gelbildende Formulierung für eine parenterale Verabreichung gestaltet ist, vorzugsweise intraossäre, periossäre, intraartikuläre, periartikuläre Verabreichung, oder für intratendinös, peritendinöse, intraligamentäre oder periligamentäre Verabreichung.

14. Eine Zusammensetzung, erzielt durch das Verfahren nach einem der vorhergehenden Ansprüche 1 bis 13.

15. Die Zusammensetzung nach Anspruch 14 zur Verwendung bei der Behandlung muskuloskelettaler Erkrankungen bei einem Subjekt, wobei die muskuloskelettale Erkrankung vorzugsweise eine Knochenerkrankung oder eine Gelenkerkrankung ist.

16. Die Zusammensetzung zur Verwendung nach Anspruch 15 bei der Behandlung von Osteoarthritis.

17. Die Zusammensetzung zur Verwendung nach Anspruch 15 bei der Behandlung oder Prävention von Rissen des kranialen Kreuzbands.

18. Die Zusammensetzung zur Verwendung nach Anspruch 15 bei der Behandlung von Tendopathien.

## Revendications

1. Un procédé de préparation d'une composition gélifiante pour une administration parentérale, ledit procédé comprend les étapes de mélange d'un mélange de plasma dérivé de mammifère comprenant des lipides et/ou des phospholipides et d'acide hyaluronique ou d'un sel ou d'un ester de celui-ci avec une solution de calcium, dans lequel la concentration de calcium dans la composition gélifiante résultante est entre 0,2 et 1,4 mg/ml et la concentration d'acide hyaluronique ou d'un sel ou d'un ester de celui-ci dans ladite composition gélifiante est entre 0,10 et 200 mg/ml.

2. Le procédé selon la revendication 1, **caractérisé en ce que** ladite solution de calcium est une solution de chlorure de calcium.

3. Le procédé selon la revendication 1 ou 2, **caractérisé en ce que** ladite solution de chlorure de calcium a un pH acide.

4. Le procédé selon l'une quelconque des revendications précédentes 1 à 3, **caractérisé en ce que** ledit mélange de plasma et d'acide hyaluronique est lyophilisé avant l'ajout de ladite solution de calcium.

5. Le procédé selon l'une quelconque des revendications précédentes 1 à 4, **caractérisé en ce que** lesdits lipides et/ou phospholipides sont présents en une concentration totale entre 0,2 et 7 mg/l.

6. Le procédé selon l'une quelconque des revendications précédentes 1 à 5, **caractérisé en ce que** lesdits lipides comprennent des lipides plasmatiques mineurs, dans lequel lesdits lipides plasmatiques mineurs sont de préférence choisis dans le groupe constitué du palmitoyl éthanolamide (PEA), du stéaroyl éthanolamide (SEA), de l'arachidonoyl éthanolamide (AEA), et dans lequel une concentration totale de lipides plasmatiques mineurs est entre 5 et 50 nmol/l.

7. Le procédé selon l'une quelconque des revendications précédentes 1 à 6, **caractérisé en ce que** ledit plasma a une concentration de globules blancs (GB) inférieure à 200 mil/l, de préférence une concentration de GB entre 3 et 145 mil/l, de manière davantage préférée entre 5 et 85 mil/l.

8. Le procédé selon l'une quelconque des revendications précédentes 1 à 7, **caractérisé en ce que** ledit plasma a une concentration de plaquettes sanguines (PS) inférieure à 50 000 mil/l, de préférence une concentration de PS entre 1 000 et 35 000 mil/l, de manière davantage préférée une concentration de PS entre 2 000 et 20 000 mil/l.

9. Le procédé selon l'une quelconque des revendications précédentes 1 à 8, **caractérisé en ce que** l'acide hyaluronique ou un sel ou un ester de celui-ci a un poids moléculaire inférieur à 1 800 kg/mol (1 800 kDa), de manière davantage préférée entre 700 et 1 600 kg/mol (700 et 1 600 kDa), de manière davantage préférée 700 et 1 000 kg/mol (700 et 1 000 kDa).

10. Le procédé selon l'une quelconque des revendications précédentes 1 à 9, **caractérisé en ce que** ledit mélange de plasma et d'acide hyaluronique comprend en outre un ou plusieurs composés pharmaceutiques choisis dans le groupe constitué d'un ingrédient pharmaceutique actif, d'un agent antibiotique, d'une composition cellulaire, d'une petite molécule organique, d'une protéine et d'un peptide.

11. Le procédé selon l'une quelconque des revendications précédentes 1 à 10, **caractérisé en ce que** ledit ingrédient pharmaceutique actif est un agoniste du récepteur adrénergique alpha-2, de préférence la clonidine.

12. Le procédé selon l'une quelconque des revendications précédentes 1 à 11, **caractérisé en ce que** ladite petite molécule organique est un composant d'échafaudage ou de matrice ayant des propriétés ostéoconductrices, de préférence des particules de phosphate tricalcique (PPT).

13. Le procédé selon l'une quelconque des revendications précédentes 1 à 12, **caractérisé en ce que** ladite formulation gélifiante est configurée pour une administration parentérale, de préférence pour une administration intra-osseuse, péri-osseuse, intra-articulaire, péri-articulaire, ou pour une administration intratendineuse, péritendineuse, intraligamentaire ou périligamentaire.

14. Une composition obtenue par le procédé selon l'une quelconque des revendications précédentes 1 à 13.

15. La composition selon la revendication 14 pour une utilisation dans le traitement de maladies musculosquelettiques chez un sujet, dans lequel la maladie musculosquelettique est de préférence une maladie osseuse ou une maladie articulaire.

16. La composition pour une utilisation selon la revendication 15 dans le traitement de l'arthrose.

17. La composition pour une utilisation selon la revendication 15 dans le traitement ou la prévention d'une rupture du ligament croisé crânien.

18. La composition pour une utilisation selon la revendication 15 dans le traitement de tendinopathies.
